(19) **Europäisches Patentamt**

**European Patent Office**

**Office européen des brevets**

(11) Publication number: **0 455 308 B1**

(12) **EUROPEAN PATENT SPECIFICATION**

(45) Date of publication of patent specification: **24.08.94**  (51) Int. Cl.⁵: **C10G  3/00**

(21) Application number: **91201048.5**

(22) Date of filing: **02.05.91**

(54) **Process for the conversion of methanol into liquid hydrocarbons.**

(30) Priority: **04.05.90 GB 9010076**

(43) Date of publication of application:
**06.11.91 Bulletin  91/45**

(45) Publication of the grant of the patent:
**24.08.94 Bulletin  94/34**

(84) Designated Contracting States:
**BE DE DK ES FR GB IT NL SE**

(56) References cited:
**EP-A- 0 220 343
FR-A- 2 370 712
GB-A- 2 153 250
GB-A- 2 161 716
US-A- 4 663 305**

(73) Proprietor: **SHELL INTERNATIONALE RE-
SEARCH MAATSCHAPPIJ B.V.
Carel van Bylandtlaan 30
NL-2596 HR Den Haag (NL)**

(72) Inventor: **Scheffer, Bob
Badhuisweg 3
NL-1031 CM Amsterdam (NL)**
Inventor: **Kortbeek, Andras Guus Theodorus
George
Badhuisweg 3
NL-1031 CM Amsterdam (NL)**

EP 0 455 308 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

**Description**

The present invention relates to a process for the conversion of methanol into liquid hydrocarbons in a catalytic reaction at elevated temperature and pressure.

The preparation of hydrocarbons from a mixture comprising hydrogen and carbon monoxide, by contacting this mixture at elevated temperature and pressure with a specific catalyst, is known in the literature as the Fischer-Tropsch hydrocarbon synthesis process. Catalysts used in the Fischer-Tropsch synthesis process usually contain one or more metals from the iron group, often together with one or more promoters, both deposited on a porous carrier material. The products that can be prepared using these catalysts usually have a very wide range of molecular weight distribution, a large variety of branched and unbranched paraffins, and often contain considerable amounts of olefins and oxygen-containing organic compounds. Usually, only a minor portion of these products comprise so-called middle distillates. The term "middle distillates" relates to hydrocarbon mixtures of which the boiling point range corresponds substantially to that of kerosine and gas oil fractions obtained in a conventional atmospheric distillation of crude mineral oil. The boiling point range of middle distillates generally lies within the range of 150 to 360 °C. The middle distillates are obtained at only relatively low yields in the Fischer-Tropsch synthesis and the pour point of the middle distillates that are obtained is unsatisfactory. Accordingly, the Fischer-Tropsch hydrocarbon synthesis process is not a very attractive route for the direct production of middle distillates on an industrial scale.

Recently, a new class of Fischer-Tropsch catalysts have been found, which catalysts yield a product in which only very minor amounts of olefins and oxygen-containing organic compounds are present and which consists substantially completely of unbranched paraffins. A considerable portion of these paraffins boil above the boiling point range of the middle distillates. It has been found that the high boiling portion of this product may be converted at high yields into middle distillates by means of hydrocracking. As a feedstock for hydrocracking at least a portion of the product is chosen having an initial boiling point lying above the final boiling point of the heaviest middle distillates desired as end product. Hydrocracking, typically proceeding at very low hydrogen consumption, yields middle distillates having a considerably better pour point than those obtained by the direct conversion of feeds using to the Fischer-Tropsch hydrocarbon synthesis process.

These novel Fischer-Tropsch catalysts belong to the group comprising cobalt as the metal component originating from the iron group, and a promoter. Suitable promoters are zirconium, titanium, chromium, ruthenium, iron, magnesium, zinc, thorium and uranium, in particular zirconium, titanium, chromium and ruthenium and mixtures thereof, more particularly zirconium and titanium.

In the preparation of hydrocarbons, mixtures of hydrogen and carbon monoxide in molar ratios varying from 0.5 to 3, are used in the Fischer-Tropsch hydrocarbon synthesis process.

Recently, it has been found that a specific type of Fischer-Tropsch catalyst may be used for the conversion of a feed comprising methanol and optionally a mixture of hydrogen and carbon monoxide into hydrocarbons. These specific catalysts, disclosed in US patents 4,568,663, 4,663,305, 4,751,345 and 4,762,954 are titania-supported cobalt catalysts, promoted with rhenium, rhenium and thorium, zirconium, hafnium, cerium or uranium.

In particular, US patent 4,663,305 discloses a zirconium, hafnium, cerium or uranium promoted cobalt catalyst and its use in a process for the conversion of methanol or syntheses gas to hydrocarbons. The promoter and the cobalt are dispersed upon a titania support. In the process, methanol, preferably with hydrogen, or synthesis gas is conducted with the promoted cobalt catalyst to yield a mixture of $C_{10}$ + linear paraffins and olefins. The process for the conversion of methanol to hydrocarbons using the promoted cobalt or titania catalyst, as exemplified in the specification of US patent 4,663,305, yields a hydrocarbon product having a $C_2$ + selectivity of from 68 to 74%wt. Cerium is disclosed as being the preferred promoter for inclusion in the a catalyst for use in the synthesis of hydrocarbons from methanol. A very similar disclosure to that of US patent 4,663,305 is made in US patent 4,762,959.

Surprisingly, it has been found during further research into Fischer-Tropsch catalysts suitable for the conversion of feeds comprising methanol into hydrocarbon mixtures suitable for the production of middle distillates, that there is another class of Fischer-Tropsch catalysts which meet the above-given requirements, which catalysts are generally used for the preparation of hydrocarbons by catalytic reaction of carbon monoxide with hydrogen. This class of Fischer-Tropsch catalyst encompasses catalysts comprising:

(i) a porous carrier material selected from the group comprising silica, alumina, and mixtures thereof;
(ii) cobalt as a metal component deposited on the porous carrier; and
(iii) a promoter selected from the group comprising zirconium, titanium, chromium, ruthenium, iron, magnesium, zinc, thorium and uranium.

Although there exists a need in the art for processes useful for the industrial conversion of methanol, optionally in combination with a mixture of hydrogen and carbon monoxide, the above-defined class of Fischer-Tropsch catalysts was clearly overlooked. It has appeared that the above-indicated class of Fischer-Tropsch catalysts is especially suitable for the preparation of very heavy hydrocarbons in high yields, which heavy hydrocarbons are very suitable for the production of middle distillates in a two-step process.

Accordingly, the present invention provides a process for the conversion of methanol into hydrocarbons, in which process a feed comprising methanol is contacted at elevated temperature and pressure with a catalyst comprising:

(i) a porous carrier material selected from the group comprising silica, alumina, and mixtures thereof;

(ii) cobalt as a metal component deposited on the porous carrier; and

(iii) a promoter selected from the group comprising zirconium, titanium, chromium, ruthenium, iron, magnesium, zinc, thorium and uranium.

The amount of cobalt and of the promoter on the porous carrier material is, among other things, dependent on the treatment by which cobalt and the promoter are deposited on the carrier material. These deposition treatments comprise precipitation, impregnation, kneading, melting, comulling and extrusion, and combinations thereof.

Generally, the amount of cobalt is 3 to 300 parts by weight per 100 parts by weight of the carrier material. When cobalt is deposited by impregnation and/or kneading, the amount of cobalt is 5 to 60, preferably 20 to 50 parts by weight per 100 parts by weight of the carrier material. However, when cobalt is incorporated into the catalyst, for example by comulling with an extrudable carrier material precursor to form an extrudable mixture, the amount of cobalt is generally 25 to 200 parts by weight, preferably 40 to 100 parts by weight, most preferably 50 to 100 parts by weight per 100 parts by weight of the carrier material.

In a similar way, the amount of promoter present in the catalyst may vary. Generally, the catalyst comprises the promoter in an amount of 0.01 to 100 parts by weight of the carrier material. If during the preparation of the catalyst first cobalt is deposited on the carrier material, and thereafter the promoter, the catalyst comprises generally 0.1 to 5, preferably 0.25 to 5 parts by weight promoter per 100 parts by weight of the carrier material. If, in the reverse mode of preparation, the promoter is deposited first on the carrier and thereafter cobalt, the promoter is generally present on the carrier material in an amount of 5 to 50, preferably 5 to 40 parts by weight per 100 parts by weight of the carrier material.

The carrier is preferably silica.

The promoter is preferably zirconium or titanium, more preferably zirconium.

With respect to the various modes of preparation which may be used to prepare the catalyst for use in the process of the present invention, reference is made to GB 2,140,701, describing that an optimum catalyst performance in terms of activity and stability may be obtained when the catalyst has L and S such as to satisfy the relation

$$(3 + 4R) > \frac{L}{S} > (0.3 + 0.4R),$$

wherein the various symbols have the following meanings:

L = the cobalt load present on the catalyst, expressed as mg Co/ml catalyst,

S = the surface area of the catalyst expressed as $m^2$/ml catalyst, and

R = the weight ratio of the amount of cobalt deposited on the catalyst by kneading to the total quantity of cobalt present in the catalyst.

The catalyst may be prepared from an extrudate of an extrudable mixture comprising a carrier material precursor and a cobalt source. The cobalt source selected for inclusion in the extrudable mixture should be such that the cobalt may be converted into the active cobalt metal component of the final catalyst. In particular, alumina may be used as the carrier material precursor. Optionally, the extrudable mixture may further comprise a promoter source, such that the promoter source may be converted to the promoter component present in the final catalyst. Suitable compounds for use as sources for the cobalt and the promoter, if present, in the extrudable mixture include oxides, hydroxides, nitrates and carbonates. If a promoter source is not included in the extrudable mixture, the promoter may be deposited as the extrudate, for example by precipitation and impregnation.

In another embodiment for the preparation of the catalyst by means of extrusion, the catalyst is formed by preparing an extrudable mixture of a carrier material precursor, a promoter source and a solvent, comulling the mixture, extruding the mixture comulled and drying the so-formed extrudate, whereafter cobalt is deposited on the extrudate, for example precipitation and impregnation.

3

The texture of the catalyst is of importance for the activity and selectivity in the catalytic conversion process of this invention. As indicated in GB 1,548,468, these catalysts have an excellent activity and selectivity if the ratio of the specific average pore diameter (p in nm) over the specific particle diameter (d in mm), is larger than 2.0. Accordingly, it is preferred to use catalysts having a p/d ratio greater than 2.0 in the process of the present invention.

The carrier material provided with both a cobalt and a promoter source is usually subjected to calcination at a temperature of 350 to 750 °C, preferably a temperature in the range of from 450 to 550 °C, in order to remove crystal water and to decompose organic and inorganic compounds to oxides and volatile decomposition products. If the calcination is carried out under specific conditions, the activity and selectivity of the catalysts may be further improved. Accordingly, the calcination is preferably performed in an atmosphere comprising nitrogen oxide, in which case the cobalt precursor is preferably cobalt nitrate. More specifically, the calcination is performed in an atmosphere containing nitrogen oxide in a concentration of at least 20% by volume, on a dry, water-free basis.

This specific calcination treatment results in the formation of cobalt oxide-containing agglomerates having a size of 1 to 10 $\mu$m.

Finally, the catalyst is activated by contacting the catalyst with hydrogen or a hydrogen-containing gas at a temperature of 200 to 350 °C. GB 2,161,716 discloses that the catalyst performance is improved when the activation is performed under a hydrogen partial pressure between 0.001 and 75 bar and during the activation the hydrogen partial pressure is increased gradually, or step-wise from an initial value $(P_{H2})_i$ to an ultimate value $(P_{H2})_u$ in such a manner as to satisfy the relation $(P_{H2})_u > 5 \times (P_{H2})_i$. Furthermore, GB 2,153,250 discloses that optimum catalyst results are obtained if the activation is performed under such conditions as to satisfy the relation:

$$\frac{D}{10^4 \times (P_{H2})^2 \times P_{Tot}} > \frac{10 \times S}{L \times (Z+1)},$$

wherein

D = space velocity, as $N1.1^{-1}.h^{-1}$,

$P_{H2}$ = hydrogen partial pressure, as bar,

$P_{Tot}$ = overall pressure, as bar,

S = surface area of the catalyst, as $m^2$/ml,

L = cobalt load of the catalyst, as mg Co/ml, and

Z = zirconium load of the catalyst, as mg Zr/100 mg carrier material

Accordingly, the aforementioned activation procedures may advantageously be applied to the catalyst used in the process of the present invention.

Suitable catalysts for the conversion of methanol comprising feeds into liquid hydrocarbons are disclosed, for example, in GB 1,548,468; GB 2,125,062; GB 2,130,113; GB 2,140,701; GB 2,153,250; GB 2,161,177; GB 2,164,266; GB 2,161,716; EP 178,008; and EP 221,598.

The catalysts are preferably used in the form of spherical, cylindrical or lobed particles having a nominal diameter of 0.5 to 5 mm, preferably 1 to 2 mm.

The preparation of hydrocarbons from a feed comprising methanol, methanol and hydrogen, or methanol and synthesis gas is performed at a temperature between 100 and 600 °C, preferably 150 to 350 °C, more preferably 180 to 270 °C, most preferably 200 to 250 °C, at a total pressure of 1 to 200 bar absolute, preferably 10 to 70 bar absolute, more preferably from 30 to 50 bar. The space velocity is about 200 to 20,000 $m^3$ (STP) gaseous feed/$m^3$ reaction zone/hour. The term "STP" as used herein refers to a standard temperature of 0 °C and a pressure of 1 bar absolute. Preferably, the feed comprises methanol and hydrogen in a molar ratio of 1:1 to 60:1, preferably 4:1 to 60:1, still more preferably 8:1 to 30:1. The feed may also comprise methanol and synthesis gas.

In general, in processes for the conversion of synthesis gas to hydrocarbon it is important, and most desirable, that the overall conversion obtained on the basis of carbon monoxide in the synthesis gas feed be as high as possible, preferably 100%. To this end, it is usual to recycle unconverted carbon monoxide and hydrogen leaving the outlet of the hydrocarbon synthesis reaction zone to the inlet of the reaction zone where, with additional fresh synthesis gas feed, the carbon monoxide and hydrogen again contact the catalyst. In one embodiment of the process of the present invention, both synthesis gas and methanol are fed to the hydrocarbon synthesis reaction zone. In this embodiment, unconverted carbon monoxide and hydrogen leaving the reaction zone are converted into methanol which is then recycled to the inlet of the reaction zone and combined with fresh synthesis gas feed. If desired, a portion of the methanol may be removed from the recycle stream as a product. In such an operation, the ratio of methanol to hydrogen entering the reaction zone may be lower than 1:1, for example ranging from 0.1:1 to 1:1, depending upon the quantity of methanol recycled.

The conversion of synthesis gas into methanol may be carried out using any of the processes well known in the art. Preferably, the synthesis gas is contacted with a catalyst system comprising oxides of one or more of zinc, chromium, or copper. Particular preference is given to catalyst systems comprising a combination of oxides of zinc and copper. The catalyst may comprise a carrier. A preferred carrier is alumina, optionally in combination with silica. The synthesis gas is contacted with the catalyst at a temperature of from 150 to 300 °C, preferably 240 to 270 °C, and at a pressure in the range of from 10 to 100 bar, preferably 30 to 70 bar. The synthesis gas may, optionally, be contacted with the catalyst in the presence of liquid methanol or a suitable solvent to dissolve the methanol. It is most convenient if the synthesis gas is contacted with the catalyst system at substantially the same pressure as that prevailing in the hydrocarbon synthesis reaction zone. Alternatively, it may be preferred to include a synthesis gas compression stage whereby the methanol synthesis stage operates at a pressure greater than that under which the hydrocarbon synthesis stage operates. The precise operating conditions of the methanol synthesis stage are well known in the art, being taught for example in the specification of US patent No. 4,520,216.

In a preferred embodiment of the process according to the present invention, the preparation of hydrocarbons from methanol is used as the first step in a two-step process for the preparation of middle distillates.

To this end the hydrocarbon product, or at least that part of the product which has an initial boiling point above the final boiling point of the desired middle distillate fraction, is subjected to a catalytic hydrotreatment as the second step in the process.

The catalytic hydrotreatment is suitably carried out by contacting the hydrocarbon material from the first step at elevated temperatures and pressures and in the presence of hydrogen with a catalyst comprising one or more metals having hydrogenation activity, supported on a carrier.

In the catalytic hydrotreatment, preference is given to the use of a catalyst comprising one or more metals from Group VIII, supported on a carrier. In particular, a catalyst is preferred comprising platinum on a carrier, 13 to 15%W of which comprises alumina and the remainder silica. The preferred reaction conditions in the hydrotreatment are temperatures in the range of 175 to 400 °C, in particular 250 to 350 C, a hydrogen partial pressure of 1 to 25 MPa, in particular 2.5 to 15 MPa, a space velocity of 0.1 to 5 kg 1 h, in particular 0.25 kg 1 h and a hydrogen/oil ratio of 100 to 5000 Nl kg, in particular 250 to 2500 Nl kg.

The present invention will be further described by way of the following illustrative example.

Example

A catalyst was prepared using a carrier comprising spherical silica particles. The carrier was dried at 120 °C. Thereafter, the carrier particles were immersed in a solution of cobalt nitrate in water. The amount of solution used was such that its volume corresponded substantially to the total pore volume of the carrier particles. The solution had a viscosity measured at 60 °C of 1.7 cS. After drying and calcining at 500 °C, the cobalt loaded silica carrier was immersed in a solution of zirconium nitrate in water. Again, the quantity of the solution used was such that the volume corresponded substantially to the total pore volume of the silica carrier particles. Finally, the cobalt and zirconium loaded carrier was dried and calcined at 500 °C.

The catalyst thus prepared was reduced (260 °C, 3 bar absolute, 6000 GHSV), in a gas stream comprising hydrogen, the hydrogen concentration being gradually increased (1 to 100%).

Thereafter, the catalyst was contacted with methanol at the following test conditions: pressure 26 bar (absolute), 210 °C, and 800 GHSV. The $C_5^+$ selectivity (%w on $C_1^+$) was more than 80%. Thus, it will be appreciated that the promoted cobalt on silica catalyst exhibited a significantly greater selectivity to the desired heavy hydrocarbons than the titania-based catalysts of the prior art.

**Claims**

1. Process for the conversion of methanol into liquid hydrocarbons, in which process a feed comprising methanol is contacted at elevated temperature and pressure with a catalyst comprising:
   (i) a porous carrier material selected from the group comprising silica, alumina, and mixtures thereof;
   (ii) cobalt as a metal component deposited on the porous carrier; and

5

(iii) a promoter selected from the group comprising zirconium, titanium, chromium, ruthenium, iron, magnesium, zinc, thorium and uranium.

2. Process as claimed in claim 1, wherein the catalyst comprises cobalt in an amount of 3 to 300 parts by weight per 100 parts by weight of the carrier material.

3. Process as claimed in either of claims 1 or 2 characterized in that the promoter is present in an amount of 0.01 to 100 parts by weight per 100 parts by weight of the carrier material.

4. Process as claimed in any preceding claim, characterized in that cobalt and/or the promoter is/are deposited on the carrier material by precipitation, impregnation, kneading, melting, comulling and/or extrusion.

5. Process as claimed in claim 4, characterized in that the cobalt is deposited on the catalyst by kneading and the catalyst has L and S such as to satisfy the relation

$$(3 + 4R) > \frac{L}{S} > (0.3 + 0.4R),$$

wherein the various symbols have the following meanings:

L = the cobalt load present on the catalyst, expressed as mg Co/ml catalyst,
S = the surface area of the catalyst expressed as $m^2$/ml catalyst, and
R = the weight ratio of the amount of cobalt deposited on the catalyst by kneading to the total quantity of cobalt present in the catalyst.

6. Process as claimed in any of claims 1 to 4, characterized in that the catalyst is prepared from an extrudate formed by preparing an extrudable mixture of a carrier material precursor, a cobalt source and a solvent, comulling the mixture, extruding the comulled mixture and drying the so formed extrudate.

7. Process as claimed in any preceding claim, characterized in that the conversion is performed at temperature of from 200 to 250 °C.

8. Process as claimed in any preceding claim, characterized in that the conversion is performed at a pressure of from 10 to 70 bar.

9. Process as claimed in any preceding claim, characterized in that the feed comprises methanol and hydrogen in a molar ratio of from 1:1 to 60:1.

10. Process as claimed in any preceding claim, characterized in that the feed comprises methanol and synthesis gas.

11. Process as claimed in claim 10, characterized in that the feed comprises methanol prepared from synthesis gas remaining after contact with the catalyst as defined in any one of claims 1 to 6.

**Patentansprüche**

1. Ein Verfahren zur Umwandlung von Methanol in flüssige Kohlenwasserstoffe, in welchem Verfahren eine Einspeisung, die Methanol enthält, bei erhöhter Temperatur und Druck mit einem Katalysator kontaktiert wird, welcher enthält:
(i) ein poröses Trägermaterial, ausgewählt aus der Gruppe, die Siliciumdioxid, Aluminiumoxid, und Mischungen davon umfasst;
(ii) Kobalt als eine Metallkomponente, welche auf dem porösen Träger niedergeschlagen ist; und
(iii) einen Promotor, welcher aus der Gruppe ausgewählt ist, die Zirkon, Titan, Chrom, Ruthenium, Eisen, Magnesium, Zink, Thorium und Uran umfasst.

2. Verfahren wie in Anspruch 1 beansprucht, wobei der Katalysator Kobalt in einer Menge von 3 bis 300 Gewichtsteilen auf 100 Gewichtsteile des Trägermaterials beinhaltet.

**3.** Verfahren wie in irgend einem der Ansprüche 1 oder 2 beansprucht, dadurch gekennzeichnet, daß der Promotor in einer Menge von 0,01 bis 100 Gewichtsteilen auf 100 Gewichtsteile des Trägermaterials anwesend ist.

**4.** Verfahren wie in irgendeinem vorhergehenden Anspruch beansprucht, dadurch gekennzeichnet, daß Kobalt und/oder der Promotor durch Ausfällung, Imprägnierung, Kneten, Schmelzen, Vermahlen und/oder Extrusion auf dem Trägermaterial niedergeschlagen wird/werden.

**5.** Verfahren wie in Anspruch 4 beansprucht, dadurch gekennzeichnet, daß das Kobalt auf dem Katalysator durch Kneten niedergeschlagen wird und der Katalysator einen Wert für L und S dergestalt aufweist, daß die Beziehung erfüllt wird:

$$(3 + 4R) > \frac{L}{S} > (0,3 + 0,4R),$$

wobei die verschiedenen Symbole die folgenden Bedeutungen haben:

L = die auf dem Katalysator anwesende Kobaltbeladung, ausgedrückt in mg Co/ml Katalysator,

S = die Oberflächengröße des Katalysators, ausgedrückt in $m^2$/ml Katalysator, und

R = das Gewichtsverhältnis der Menge des durch Kneten auf dem Katalysator niedergeschlagenen Kobalts zur Gesamtmenge des im Katalysator anwesenden Kobalts.

**6.** Verfahren wie in irgend einem der Ansprüche 1 bis 4 beansprucht, dadurch gekennzeichnet, daß der Katalysator aus einem Extrudat hergestellt worden ist, gebildet durch Herstellung einer extrudierbaren Mischung aus einem Trägermaterialvorläufer, einer Kobaltquelle und einem Lösungsmittel, Vermahlen der Mischung, Extrudieren der vermahlenen Mischung und Trocknen des so gebildeten Extrudats.

**7.** Verfahren wie in irgendeinem vorhergehenden Anspruch beansprucht, dadurch gekennzeichnet, daß die Umwandlung bei einer Temperatur von 200 bis 250 °C durchgeführt wird.

**8.** Verfahren wie in irgendeinem vorhergehenden Anspruch beansprucht, dadurch gekennzeichnet, daß die Umwandlung bei einem Druck von 10 bis 70 bar durchgeführt wird.

**9.** Verfahren wie in irgendeinem vorhergehenden Anspruch beansprucht, dadurch gekennzeichnet, daß die Einspeisung Methanol und Wasserstoff in einem molaren Verhältnis von 1:1 bis 60:1 beinhaltet.

**10.** Verfahren wie in irgendeinem vorhergehenden Anspruch beansprucht, dadurch gekennzeichnet, daß die Einspeisung Methanol und Synthesegas beinhaltet.

**11.** Verfahren wie in Anspruch 10 beansprucht, dadurch gekennzeichnet, daß die Einspeisung Methanol beinhaltet, hergestellt aus Synthesegas, welches nach dem Kontakt mit dem Katalysator, wie definiert in irgend einem der Ansprüche 1 bis 6, zurückbleibt.

**Revendications**

**1.** Procédé pour la conversion de méthanol en hydrocarbures liquides, dans lequel une charge comprenant du méthanol est mise en contact à température et pression élevées avec un catalyseur comprenant :

(1) une matière poreuse de support choisie dans le groupe comprenant la silice, l'alumine et leurs mélanges ;

(2) du cobalt comme constituant métallique déposé sur le support poreux ; et

(3) un promoteur choisi dans le groupe comprenant le zirconium, le titane, le chrome, le ruthénium, le fer, le magnésium, le zinc, le thorium et l'uranium.

**2.** Procédé selon la revendication 1, dans lequel le catalyseur comprend le cobalt à raison d'une quantité de 3 à 300 parties en poids pour 100 parties en poids de la matière de support.

**3.** Procédé selon l'une des revendications 1 ou 2, caractérisé en ce que le promoteur est présent à raison d'une quantité de 0,01 à 100 parties en poids pour 100 parties en poids de la matière de support.

4. Procédé selon l'une quelconque des revendications précédentes, caractérisé en ce que le cobalt et/ou le promoteur sont déposés sur la matière de support par précipitation, imprégnation, malaxage, fusion, comalaxage et/ou extrusion.

5. Procédé selon la revendication 4, caractérisé en ce que le cobalt est déposé sur le catalyseur par malaxage et le catalyseur à des valeurs de L et S satisfaisant la relation

$$(3 + 4R) > \frac{L}{S} > (0,3 + 0,4 R),$$

où les diverses lettres ont les significations suivantes :

L = la charge de cobalt présente sur le catalyseur, exprimée en mg de Co par ml de catalyseur,

S = la surface spécifique du catalyseur, exprimée en $m^2/ml$ de catalyseur, et

R = le rapport en poids de la quantité de cobalt déposée sur le catalyseur par malaxage à la quantité totale de cobalt présente dans le catalyseur.

6. Procédé selon l'une quelconque des revendications 1 à 4, caractérisé en ce que le catalyseur est préparé à partir d'un extrudat formé en préparant un mélange extrudable d'un précurseur de matière de support, d'une source de cobalt et d'un solvant, en comalaxant le mélange, en extrudant le mélange comalaxé et en séchant l'extrudat ainsi formé.

7. Procédé selon l'une quelconque des revendications précédentes, caractérisé en ce que la conversion est effectuée à une température de 200 à 250°C.

8. Procédé selon l'une quelconque des revendications précédentes, caractérisé en ce que la conversion est effectuée à une pression de 10 à 70 bars.

9. Procédé selon l'une quelconque des revendications précédentes, caractérisé en ce que la charge comprend du méthanol et de l'hydrogène dans un rapport molaire compris entre 1:1 et 60:1.

10. Procédé selon l'une quelconque des revendications précédentes, caractérisé en ce que la charge comprend du méthanol et du gaz de synthèse.

11. Procédé selon la revendication 10, caractérisé en ce que la charge comprend du méthanol préparé à partir du gaz de synthèse restant après contact avec le catalyseur comme défini dans l'une quelconque des revendications 1 à 6.